# EUROPEAN PATENT APPLICATION

(11) **EP 1 270 021 A1**
(43) Date of publication of application: **02.01.2003**
(21) Application number: 02012808.8
(22) Date of filing: 10.06.2002
(51) Int. Cl.: A61L 9/03, A61L 9/12, A01M 1/20, A01M 13/00, B60H 3/00

(54) **Refill for the diffusion of solutions and related diffusing device**

(30) Priority: 20.06.2001 IT MI20011296
(71) Applicant: ZOBELE HOLDING S.P.A., 38100 TRENTO (IT)
(72) Inventor: Zobele, Franco, 38100 Trento (IT); Marchetti, Fabio, 38050 Povo (Trento) (IT)
(74) Representative: Petruzziello, Aldo

(57) **Abstract**

A refill (1) for the diffusion of solutions comprises a shell (2) made up of two half-shells (4, 5) that may be fitted together to form a chamber (7) to contain the solution, and a wick (3) contained inside the shell (2) made up of a stem part (12) placed in the chamber (7) containing the solution and an upper part (11), at least partially in contact with the exterior, to diffuse the solution.

## Description

This invention refers to a refill for the diffusion of solutions, in particular a refill with an evaporation wick for the diffusion of solutions containing active principles such as deodorants, insecticides, disinfectants and similar substances.

The refill according to the invention, depending upon the solutions it contains, may operate on its own, for example with a stand that keeps it vertical, or attached to a specially designed diffusing device, such as those devices designed to cause the evaporation of the solution by heating and/or ventilation, or placed in front of a ventilation outlet, for example in a motor vehicle, or in front of a convector heater.

This invention additionally refers to a diffuser device, of the type fitted with a fan, to which the refill according to the invention may be attached.

Several kinds of refill are currently sold on the market.

Such refills generally consist of bottles or containers containing the solution, a wick used to diffuse the solution and a special wick-holding stopper to be placed on the bottle. There are also known refills in which, in place of this special stopper, the upper part of the bottle is itself designed to hold the wick and permit diffusion of the solution.

However, refills manufactured according to known art are rather complex, both with regard to the number of components to be assembled and to the structure of such components. This leads to high production costs for the refills which have repercussions upon the end product.

An objective of this invention is to overcome the drawbacks of known art, providing a refill for the diffusion of solutions that is economical and easy to manufacture.

Another objective of this invention is to provide such a refill for the diffusion of solutions made up of a limited number of component parts, with simplified structures and easily assembled with one another.

A further objective of this invention is to provide such a refill for the diffusion of solutions that is versatile and suitable for use both on its own or in combination with diffusion devices.

These objectives are achieved in accordance with the invention with the characteristics listed in the appended independent claim 1.

Another objective of this invention is to provide a diffusion device of the type fitted with a fan particularly suited to house a refill according to the invention.

This objective is achieved in accordance with the invention with the characteristics listed in the appended claim 16.

Advantageous embodiments of the invention are apparent from the dependent claims.

The refill for the diffusion of solutions, according to the invention, comprises a shell consisting of two half-shells which can be coupled together to form a reservoir chamber for the solution, and a wick contained within the shell and comprising a stem shaped part placed in the reservoir chamber for the solution and a head part, at least partially in communication with the exterior of the refill, to diffuse the solution.

The structure and the manufacture of the refill according to the invention are very simple. In fact the refill comprises only two half-shells and a wick and does not include bottles, caps and wick holders like the refills currently in use.

The two half-shells may be advantageously made in plastic by thermoforming and joined together by heat sealing or by high frequency or ultrasound welding. In this way, both the material used and the refill production process prove to be extremely economical.

Further characteristics of the invention will be made clearer from the detailed description that follows, referring to purely exemplary and therefore non-limiting embodiments thereof, illustrated in the appended drawings, in which:

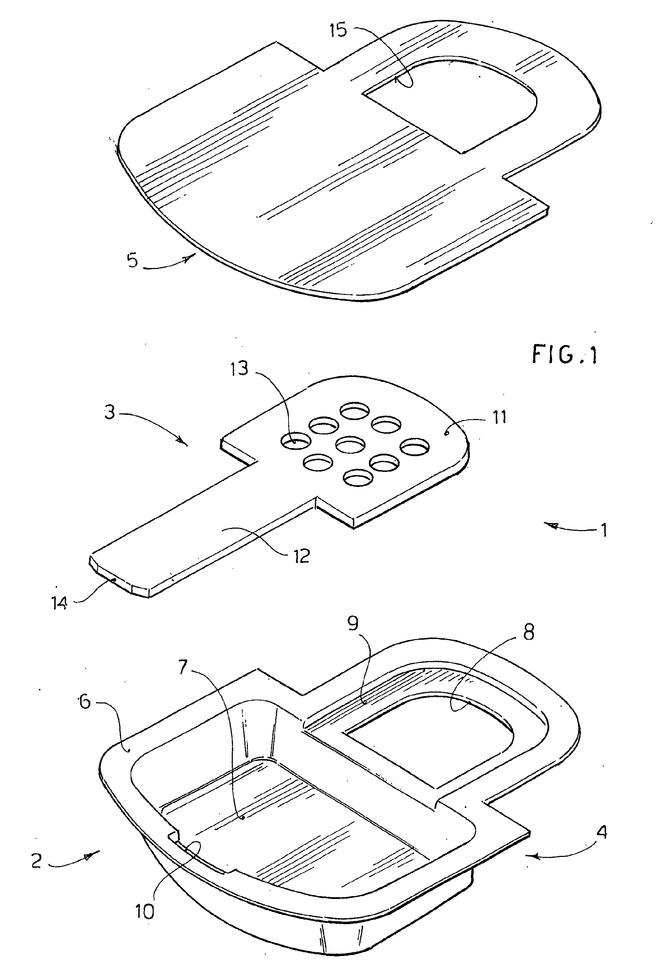

Fig. 1 is an exploded perspective view of a first embodiment of the refill for the diffusion of solutions according to the invention;

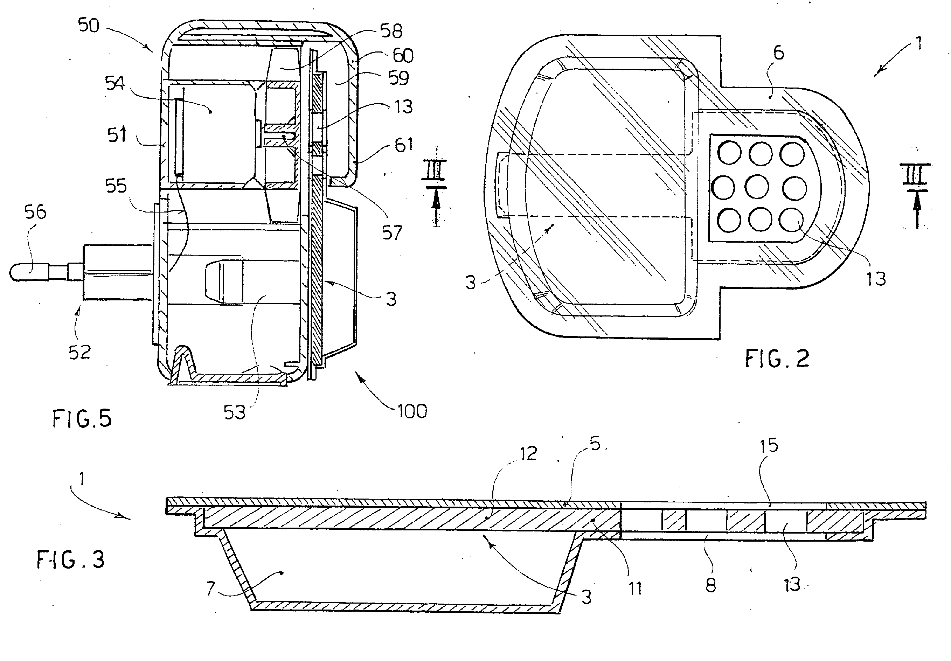

Fig. 2 is a top plan view of the refill in Fig. 1 when assembled;
Fig. 3 is an enlarged sectional view of the refill according to the invention taken along the section plane III-III of Fig. 2;
Fig. 4 is an exploded top plan view showing the refill in a second embodiment of the invention;
Fig. 5 is a partially sectional view, showing the refill in Fig. 4 assembled and fitted on a fan-type apparatus according to the invention;
Fig. 6 is a perspective view of a stand for the refill according to the invention;
Fig. 7 is a partially sectional view showing a refill according to the invention fitted onto the stand in Fig. 6.

The refill for the diffusion of solutions, indicated as a whole with the reference number 1, is described with the aid of the figures.

The refill 1 comprises a shell or valve 2 containing the solution and a wick 3 for the diffusion of the solution.

The shell 2 is consists of a front half-shell or half-valve 4 designed to contain the solution and a rear half-shell or half-valve 5 serving as a cover which can be coupled with the front half-shell 4.

The front half-shell 4 has a flat peripheral flange 6 which defines a recessed chamber or reservoir 7 to contain the solution. Next to the chamber 7 there is a substantially rectangular opening 8. Around the opening 8 there is a substantially circular or annular recess 9, slightly deeper than the peripheral flange 6.

In the wall of chamber 7 opposite the wall that separates it from opening 8 there is a slot 10 to hold the wick 3 in position.

The wick 3 is in the shape of a substantially rectangular plate with a wider head part 11 and a narrower stem-shaped part 12. The head part 11 of the wick 3 is shaped in such a way that it will fit into the recess 9 in the front half-shell 4. A series of holes 13 have been made in the head part 11 of the wick to improve evaporation of the solution.

When the head part 11 of the wick is positioned in the recess 9 of the front half-shell 4, the series of holes 13 is surrounded by the opening 8 so that they are in direct contact with the exterior. The figures show nine holes arranged in rows of three purely as an example, but the wick may be made with a different number of holes or without any holes at all.

The stem 12 of the wick 3 is long enough to span the whole width of the chamber 7 in the front half-shell 4. The stem 12 has a free end 14 designed to fit into the slot 10 in the front half-shell.

The rear half-shell or cover 5 is shaped like a flat plate with the same outer profile as the front half-shell 4. The rear half-shell 5 has an opening 15 designed to match the opening 8 when the rear half-shell is placed on top of the front half-shell. In this way, the holes 13 in the wick are in contact with the exterior of the refill from the side corresponding to the rear half-shell 5 too.

The opening 15 in the rear half-shell 5 therefore permits the passage of air through the holes 13 and is suitable for use, in particular, when the refill 1 uses ventilation, for example in combination with a fan or the ventilator outlets inside a motor vehicle. However, the opening 15 in the rear half-shell 5 may also be omitted, for example when the refill 1 is used with devices for heating by contact.

Assembly of the refill 1 is extremely simple. In fact, after filling the chamber 7 with liquid solution, the upper part 11 of the wick 3 must be placed in the recess 9 in the front half-shell 4 and the free end 14 of the stem 12 of the wick must be placed in the slot 10 in the front half-shell 4, so that the stem 12 is soaked with the liquid within the chamber 7.

The rear half-shell 5 must then be coupled to the front half-shell 4 in order to hermetically seal the chamber 7 and hold the wick 3 sandwiched between the two half-shells 4 and 5. In this way, the group of holes 13 in the wick remain uncovered because they are contained inside the openings 8 and 15 in the two half-shells 4 and 5. The liquid substance absorbed by the wick 3 can thus evaporate from the surface of the upper part 11 of the wick through the holes 13.

The wick 3 must be made in a suitable material that has the capillarity and the porosity required to transport the liquid contained in chamber 7 from the stem 12 to the head part 11, which forms the area of evaporation, thus permitting the evaporation of the liquid from the head part 11. For this purpose the wick may be made in a blend of cellulose fibre and cotton or may be made in any other kind of porous material such as ceramic, polyethylene fibre, polypropylene fibre or similar material. Preferably the wick 3 should be made in materials suitable for welding with the materials in which the half-shells 4 and 5 are made. In fact, the wick may be welded to the half-shells by means of methods such as heat sealing, ultrasound or high frequency welding.

The two half-shells 4 and 5 may be made in any material compatible with the liquid solution that they must contain. Nevertheless, it is preferable that the two half-shells 4 and 5 should be made in plastic material. In this case, the front half-shell 4 is made by thermoforming to shape the chamber 7 and the recess 9. The openings 8 and 15 respectively in the front half-shell 4 and the rear half-shell 5 are made by die cutting.

If weldable plastic material is used, the rear half-shell 5 may be welded to the front half-shell 4 along the peripheral flange 6. The wick 3, at least around the perimeter of the head part 11 is welded to the two half-shells 4 and 5 to prevent the liquid contained in the chamber 7 from leaking out when the refill 1 is not in an upright position. Then to ensure that the refill is tightly sealed, the two half-shells 4 and 5 are welded together along their peripheral edge and welded to the wick 3 around the evaporation area where the head part 11 of the wick 3 is in contact with the air.

This solution involving the welding of the wick 3 to the half-shells 4 and 5 has a dual purpose: that of ensuring that the refill 1 is tightly sealed and of holding the wick 3, keeping it taut. This prevents the wick from folding or bending during use with the

result that the wick is heated more uniformly permitting better evaporation of the solution from the wick.

The material of the two half-shells 4 and 5 must be such that they can be welded to one another as well as to the wick.. For this purpose, for example, PVC (polyvinyl chloride) treated with polythene may be used or another material that is compatible with the liquid contained in the reservoir and that can be treated with glue that reacts to heat so that it welds both with the half-shells 4 and 5 and with the wick 3. For example, the two half-shells 4 and 5 may be made in PET (polyethylene terephthalate) and the wick 3 may be made in porous PET or the two half-shells 4 and 5 may be made in PP (polypropylene) and the wick 3 in porous PP.

Once the refill 1 has been assembled, the opening 8 and 15 in the half-shells 4 and 5 are covered with two strips which may be removed by the user, for example with two self-adhesive labels, in order to ensure that the refill remains tightly sealed before use and to permit subsequent re-sealing of the refill, should it be necessary to transfer the refill elsewhere or interrupt its operation.

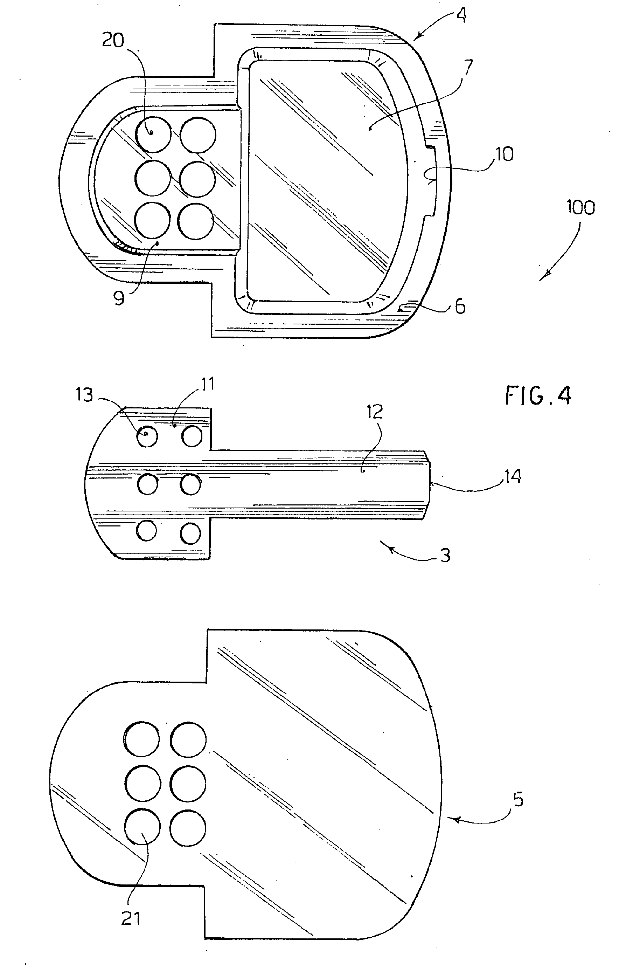

Fig. 4 shows a second embodiment of the refill according to the invention in which equal elements or elements corresponding to those already described in the first embodiment are indicated with the same reference numbers.

In this second embodiment, the refill is indicated with the reference number 100 and differs from refill 1 in the first embodiment only due to the fact that in the front half-shell 4 and the rear half-shell 5, in place of opening 8 and 9, a series of holes 20 and 21 are provided. The two series of holes 20 and 21, respectively in the front half-shell 4 and the rear half-shell 5, match the holes 13 in wick 3, when the head part 11 of the wick is inserted into the recess 9 in the front half-shell 4 and the rear half-shell 5 is placed on top of the front half-shell. The diameter of each hole in the series of holes 20 and 21 may be slightly larger than the diameter of the holes 13 in the wick, to provide a larger evaporation surface in the wick in communication with the exterior.

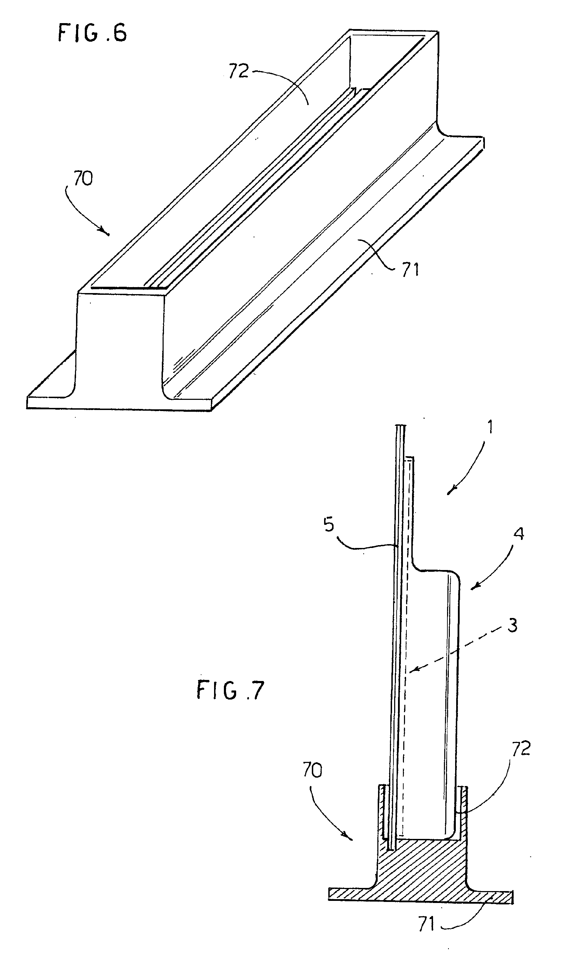

As shown in Fig. 5, the refill 100 is particularly suited to application on a fan-type diffusing device 50.

The diffusing device 50 comprises a housing 51 incorporating an electric plug 52 on the back to be inserted into a power socket. The electric plug 52 is mounted to revolve on a pivot 53 provided in the housing 51 of the diffusing device 50. In this way, the diffusing device 50 may be turned on the electric plug 52 according to the positioning of the power socket into which the electric plug 52 is inserted.

Inside the housing 51 there is an electric motor 54 which receives power from the power cables 55 connected to the electric contacts of poles 56 of the electric plug 52. A fan 58 is connected to the shaft 57 of the electric motor 54. In this way, when the plug 52 is inserted into the mains supply, the motor 54 is powered and turns fan 58.

The front of the fan 58 faces a chamber 59 provided inside the housing 51 and delimited by a front wall 60 in which slits 61 are provided to let air through. The refill 100 is attached to the diffusing device 50, so that the part of the wick 3 in which the holes 13 are made fits into chamber 59.

In this way, when the fan 58 turns, the air flow generated by it blows through the holes 21 in the rear half-shell 5, the holes 13 in the wick 3 and the holes 20 in the front half-shell and lastly leaves the chamber 59 through the slits 61 in the front wall 60 of the diffusing device 50. The air flow generated by the fan 58 therefore facilitates the evaporation of the solution that is in the area of the wick surrounding the holes 13.

Figures 6 and 7 show a stand 70 suited to keeping the refill 1 in an upright position. The support 70 comprises a base 71 designed to be placed on a flat surface, such as a low table or a shelf, for example, and a slot 72 open at the top designed to hold the lower part of the refill 1.

In this way the head part 11 of the wick 3 is at the top, facilitating the evaporation of the liquid and the chamber 7 containing the liquid is at the bottom limiting consumption of the liquid. The refill 1 with the related stand 70 may be positioned in any place where the diffusion of the essence is required and the refill 1 can operate on its own and may be placed in front of a ventilation outlet or a convector heater.

Numerous variations and modifications of detail within the reach of a person skilled in the art may be made to these embodiments of the invention without departing from the scope of the invention expressed by the appended claims.

## Claims

1. Refill (1; 100) for the diffusion of solutions comprising:
- a shell (2) consisting of a front half-shell (4) and a rear half-shell (5) which can be coupled together to form a chamber (7) to contain the said solution to be diffused,
- a wick (3) contained inside the said shell (2) comprising a stem part (12) placed in the said chamber (7) containing the solution and a head part (11) designed to be at least partially in contact with the exterior, to diffuse the said solution,
- said front half-shell (4) comprising a flat peripheral flange (6) inside which there is a recessed chamber (7) to contain the solution and a recess (9) to hold the head part (11) of the said wick,
- said rear half-shell (5) shaped like a flat plate with substantially the same outer profile as the said front half-shell (4) in order that it may be applied to the peripheral flange (6) of the said front half-shell (4),
**characterised by** the fact that in the said recess (9) that holds the said head part (11) of the said wick and/or in the said rear half-shell (5) at least one opening (8; 20) and/or (15; 21) are provided to ensure that the head part (11) of the wick is at least partially in contact with the exterior.

2. Refill according to claim 1, **characterised by** the fact that the said wick (3) is glued or heat sealed to the said half-shells (4, 5) to provide a better seal and to keep the wick (3) taut.

3. Refill according to claim 1 or 2, **characterised by** the fact that in the upper part (11) of the said wick (3) a series of holes (13) are provided communicating with the exterior to increase the evaporation surface of the said wick.

4. Refill according to claim 3, **characterised by** the fact that at least one of the said openings (8) in the front half-shell is a substantially rectangular opening of such a size that the said holes (13) in the wick (3) may be in communication with the exterior.

5. Refill according to claim 3, **characterised by** the fact that at least one of the said openings (20) of the front half-shell comprises a series of holes (20) arranged in register with the said holes (13) in the head part of the wick.

6. Refill (100) according to any one of claims 3 to 5, **characterised by** the fact that at least one of the said openings (15) in the rear half-shell is a substantially rectangular opening of such a size that it allows the said holes (13) in the wick (3) to communicate with the exterior.

7. Refill (100) according to any one of claims 3 to 5, **characterised by** the fact that at least one of the said openings (21) in the rear half-shell comprises a series of holes (21) arranged in register with said holes (13) in the head part of the wick.

8. Refill according to any one of the previous claims, **characterised by** the fact that at least one of the said openings (8; 20) in the front half-shell (4) and the said openings (15; 21) in the rear half-shell is closed by a strip which can be removed by the user.

9. Refill according to any one of the previous claims, **characterised by** the fact that in a wall of the recessed chamber (7) in the front half-shell (4) a slot is provided into which the end (14) of the stem (12) of the wick opposite the head part (11) of the wick may be inserted to hold the wick (3) firmly in place.

10. Refill according to any one of the previous claims, **characterised by** the fact that the said wick is made in porous material such as a blend of cellulose fibre and cotton, or ceramic, or polyethylene fibre, or polypropylene fibre.

11. Refill according to any one of the previous claims, **characterised by** the fact that the said shell (4) is made in plastic material.

12. Refill according to claim 11, **characterised by** the fact that the said shell is made in PVC (polyvinyl chloride) treated with polythene.

13. Refill according to claim 11 or 12, **characterised by** the fact that the said front half-shell (4) is made in thermoformed plastic material.

14. Refill according to any one of claims 11 to 13, **characterised by** the fact that the said front half-shell (4) and the said rear half-shell (5) are welded together by heat sealing.

15. Refill according to any one of the previous claims, **characterised by** the fact that it comprises a stand (70) designed to permit placing of the refill in an upright position on a flat surface.

16. Diffusing device (50) comprising:
- an electric plug (52) suitable for insertion into a mains power socket,
- an electric motor (54) connected electrically to the electric plug,
- a fan (58) turned by the said electric motor,
**characterised by** the fact that a refill (100) according to any one of the previous claims is fitted to the said device (50).

17. Device according to claim 16, **characterised by** the fact that the said refill (100) comprises a wick (3) sandwiched between a rear half-shell (5) and a front half-shell (4), the said wick comprising an upper part (11) with a series of holes (13), in register with the holes (20, 21) provided respectively in the front and rear half-shells, the said fan (58) with the front facing a chamber (59) inside the housing of the said device (50) in which the part of the refill (100) containing the head part (11) of the wick is positioned, so that the air flow generated by the fan may blow through the holes (21) in the rear half-shell, the holes (13) in the wick and the holes (20) in the front half-shell.
